Europäisches Patentamt

European Patent Office

Office européen des brevets

(11) Veröffentlichungsnummer: **0 228 506**
**A2**

(12) **EUROPÄISCHE PATENTANMELDUNG**

(21) Anmeldenummer: **86112373.5**

(22) Anmeldetag: **06.09.86**

(51) Int. Cl.⁴: **A61B 1/06** , A61B 17/36 , G02B 6/36

(30) Priorität: **08.11.85 DE 3539638**

(43) Veröffentlichungstag der Anmeldung:
**15.07.87 Patentblatt 87/29**

(84) Benannte Vertragsstaaten:
**AT BE CH DE FR GB IT LI LU NL SE**

(71) Anmelder: **Messerschmitt-Bölkow-Blohm Gesellschaft mit beschränkter Haftung Robert-Koch-Strasse D-8012 Ottobrunn(DE)**

(72) Erfinder: **Hahn, Andreas Ringbergstrasse 19 D-8029 Sauerlach(DE)**
Erfinder: **Rottmann, Alfons Goethering 40 D-8018 Grafing(DE)**

(54) **Lichtleiter, insbesondere für medizinische Instrumente.**

(57) Die Erfindung bezieht sich auf einen Lichtleiter - (I), insbesondere für medizinische Instrumente mit einer Lichtleitfaser (3), die gegebenenfalls mit einem Schutzmantel (I3) umgeben ist, einer die Lichtleitfaser mit Abstand umgebenden Hülle (4) aus einem oder mehreren Schläuchen (5, 6) und einem Einkoppelstück (2) auf einer Seite des Lichtleiters (I) zum Einkoppeln von Lichtstrahlung in die Lichtleitfaser - (3). Die Lichtleitfaser (3) und ein Schlauch (5, 6) der Hülle (4) sind in dem Einkoppelstück (2) eingespannt, während das andere Ende der Lichtleitfaser in dem Schlauch (5) der Hülle (4) eingespannt ist. Um bei Abbiegungen, insbesondere beim Aufwickeln der Lichtleitfaser (3), in Längsrichtung wirkende Spannungen auf Hülle (4) und Lichtleitfaser (3) zu verhindern, wird gemäß der Erfindung in die Hülle - (4) ein axial längenveränderliches, radial jedoch im wesentlichen nicht dehnbares Längenausgleichselement (20, 20a bis 20d) eingesetzt.

FIG.1

## Lichtleiter, insbesondere für medizinische Instrumente

Die Erfindung bezieht sich auf einen Lichtleiter gemäß dem Oberbegriff des Patentanspruches I.

Lichtleiter werden in der Medizin z.B. für Laseroperationsinstrumente oder für Endoskope verwendet. Der Lichtleiter besteht dabei aus einem Einkoppelstück, einer Lichtleitfaser und einem diese mit Abstand umgebenden Hüllschlauch. Die Lichtleitfaser selbst besteht aus dem Lichtleitkern, z.B. aus Quarzglas, einer Silikonschicht um den Lichtleitkern und einem Mantel aus Polytetrafluorethylen.

Das Einkoppelstück dient dazu, Licht-oder Laserstrahlung fokussiert in die Lichtleitfaser einzukoppeln. Lichtleitfaser und Hüllschlauch sind hierzu in dem Einkoppelstück eingespannt. An dem anderen Ende des Lichtleiters ist die Lichtleitfaser direkt in den Hüllschlauch eingespannt.

Der Hüllschlauch ist je nach Verwendungszweck unterschiedlich aufgebaut. Er kann z.B. ein relativ dünner flexibler gas-oder flüssigkeitsdichter Schlauch sein, durch den vom Einkoppelstück Gas oder Flüssigkeit bis an das vordere Ende der Lichtleitfaser geleitet wird und dieses Ende kühlt. Derartige Lichtleiter sind für den Einsatz in flexiblen oder starren Instrumenten geeignet.

Es sind auch relativ starre und voluminöse Hüllen bekannt, die gegebenenfalls aus mehreren Schläuchen bestehen, z.B. einem gasdichten Schlauch und einem diesen umgebenden Schutzschlauch mit relativ dicker Wand. Dieses Hüllschlauchsystem dient als Bruch-oder Verletzungsschutz für den frei verlaufenden oder in ein starres Instrument eingebauten Lichtleiter. Gas-und Flüssigkeitsschutz ist für den äußeren Schutzschlauch allgemein nicht gefordert.

Bei Biegungen des Lichtleiters treten zwischen den einzelnen Teilen des Lichtleiters Spannungen auf, die Zugkräfte in Längsrichtung auf die Lichtleitfaser und deren in Längsrichtung starren Lichtleitkern bewirken. Insbesondere, wenn der bis zu drei Meter lange Lichtleiter zum Transport oder bei sonstigem Nichtgebrauch aufgewickelt wird, können diese Spannungen wegen der unterschiedlichen Radien von Hüllschlauch bzw. Hüllschlauchsystem und Lichtleiterfaser mit Lichtleitkern so groß werden, daß die Lichtleitfaser oder der Lichtleitkern in einer der Einspannstellen verschoben wird. Erfolgt dies an der Einspannstelle innerhalb des Einkoppelstücks, so ist die Fokussierung der einzukoppelnden Lichtstrahlung gestört. Außerdem kann die Lichtleitfaser oder der Lichtleitkern thermisch beschädigt werden. Wird die Lichtleitfaser oder der Lichtleitkern an der anderen Einspannstelle verschoben, so kann die Lichtleitfaser bzw. der Lichtleitkern dort leicht verletzt werden.

Falls außerdem der Lichtleitkern mit Gas oder Flüssigkeit gekühlt wird, so wird durch die Verschiebung die Kühlung abgeschwächt, der Lichtleitkern übermäßig erhitzt und gegebenenfalls unbrauchbar.

Man könnte zwar daran denken, die Lichtleitfaser mit dem Lichtleitkern mit höherer Kraft einzuspannen, um die Längsverschiebung des Lichtleitkerns zu verhindern. Dies ist jedoch nur bedingt möglich, wenn nicht die optischen Eigenschaften der Lichtleitfaser durch den Druck beeinträchtigt werden sollen. So sollte z.B. die zwischen Lichtleitkern und Mantel aus Polytetrafluorethylen eingefügte weiche Silikonschicht nicht beschädigt werden, da diese als lichtbrechende Sperrschicht für die Lichtstrahlen innerhalb des Lichtleitkerns dient. Schon dies verhindert eine beliebige Erhöhung der Einspannkraft.

Der Erfindung liegt die Aufgabe zugrunde, einen Lichtleiter der in Rede stehenden Art anzugeben, bei dem die bei Abbiegungen des Lichtleiters auftretenden axialen Spannungen zwischen einzelnen in Längsrichtung verlaufenden Elementen des Lichtleiters auf einfache Weise abgebaut werden.

Diese Aufgabe ist gemäß der Erfindung durch die im kennzeichnenden Teil des Patentanspruches I angegebenen Merkmale gelöst.

Durch die Einfügung eines Längenausgleichselementes in den Hüllschlauch des Lichtleiters wird beim Abbiegen des Lichtleiters die ansonsten entstehende Längenänderung des Hüllschlauches bzw. mehrerer Schutzschläuche gegenüber dem Lichtleitkern ausgeglichen und eine Kraftwirkung auf die Lichtleitfaser und den Lichtleitkern in deren Längsrichtung vermieden. Damit ist jedoch auch die Gefahr unterbunden, daß die Lichtleitfaser oder der Lichtleitkern an den Einspannungen verschoben wird oder aus dem umgebenden Mantel austritt.

Als Längenausgleichselement ist ein elastischer Schlauch, z.B. aus Silikon denkbar, der einige wenige Zentimeter lang ist und innerhalb des Einkoppelstückes als Teil des Hüllschlauches mit diesem verbunden ist. Damit sich dieser elastische Schlauch etwa bei Durchleiten von Gas durch den Hüllschlauch nicht radial ausdehnt und damit erneut Zugspannungen auf die Lichtleitfaser ausübt, wird er von einer kleinen Spiralfeder umgeben, die axiale Längenänderungen mitmacht, radiale Ausbeulungen jedoch verhindert.

Als Längenausgleichselement sind auch Wellschläuche aus Metall oder Kunststoff möglich, wobei im letzteren Fall der Kunststoff mit Fäden oder Geweben verstärkt sein kann, um eine radiale Dehnung bzw. Ausbeulung des Wellschlauchs zu vermeiden.

Die angegebenen Ausführungsformen für ein Längenausgleichselement können in Verbindung mit Hüllschläuchen verwendet werden, die gas- oder flüssigkeitsdicht sein sollen. Besteht diese Forderung nicht, z.B. bei den oben erwähnten Schutzschläuchen gegen mechanische Verletzung des Lichtleiters, dann kann als Längenausgleichselement in den Schutzschlauch ein Teleskopstück integriert sein, in dem der Schutzschlauch längsverschieblich gelagert ist. Dieses Teleskopstück ist bevorzugt wiederum im Bereich des Einkoppelstückes angeordnet.

Weitere Ausführungsformen der Erfindung gehen aus den Unteransprüchen hervor. Die Erfindung ist in Ausführungsbeispielen anhand der Zeichnung näher erläutert. In der Zeichnung stellen dar:

Figur I einen Lichtleiter mit einem Einkoppelstück, einer Lichtleitfaser und einem Hüllschlauch sowie einem Längenausgleichselement gemäß der Erfindung;

Figuren 2 und 3 jeweils eine geschnittene Darstellung des Lichtleiters an den Einspannstellen der Lichtleitfaser im Einkoppelstück bzw. im Hüllschlauch;

Figuren 4, 5 und 6 jeweils unterschiedliche Ausführungsformen eines Längenausgleichselementes gemäß der Erfindung für einen gasdichten Hüllschlauch;

Figur 7 eine weitere Ausführungsform eines Längenaus gleichselements für einen Hüllschlauch, der im wesentlichen als Knickschutz für die Lichtleitfaser dient;

Figur 8 einen schematischen Querschnitt längs VIII-VIII in Figur I durch eine Lichtleitfaser mit Hüllschlauch.

Ein in Figur I dargestellter Lichtleiter I weist ein Einkoppelstück 2, eine Lichtleitfaser 3 und eine Hülle 4 aus einem inneren Hüllschlauch 5 und einem äußeren Hüllschlauch 6 auf. Der innere Hüllschlauch 5 erstreckt sich über die gesamte Länge der Lichtleitfaser und dient als Gasschlauch. Der äußere Hüllschlauch 6 erstreckt sich nur über einen Teil der Länge der Lichtleitfaser und dient im wesentlichen dazu, diese vor Knicken oder anderen mechanischen Beschädigungen zu schützen.

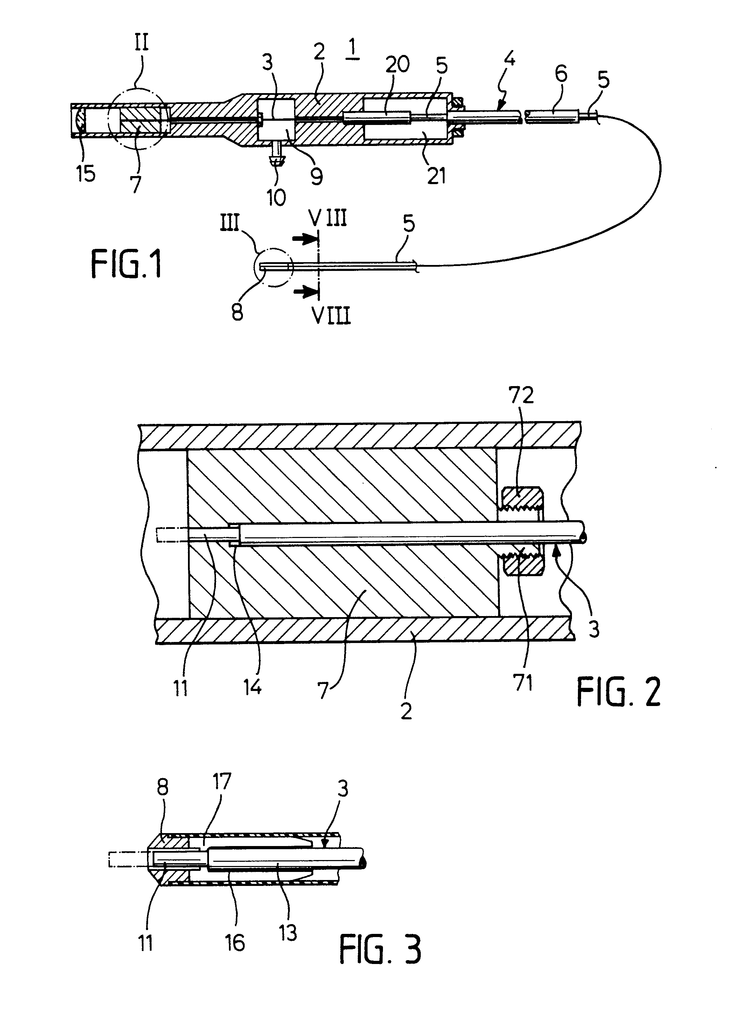

Die Lichtleitfaser 3 ist, wie näher in Figur 2 gezeigt, innerhalb des Einkoppelstückes 2 in einem Befestigungsblock 7 mit Hilfe einer die Lichtleitfa-ser 3 umgebenden Spreizhülse 71 und einer Überwurf-Spannmutter 72 eingespannt. Am anderen Ende der etwa drei Meter langen Lichtleitfaser 3 ist diese, wie näher in Figur 3 gezeigt, mit Hilfe eines Einsatzstückes 8 in dem Gasschlauch 5 eingespannt. Der Gasschlauch 5 mündet innerhalb des Einkoppelstückes 2 in einen Gasraum 9 und ist in diesem Bereich mit dem Gehäuse des Einkoppelstückes 2 verklebt. Der Gasraum ist mit einem Außenanschluß I0 versehen, über den ein Kühlgas in den Gasraum 9 und von dort in den Gasschlauch 5 eingeleitet werden kann. Dieses Gas dient zur Kühlung der Spitze der Lichtleitfaser 3 innerhalb des Einsatzstückes 8 , wie weiter unten näher beschrieben.

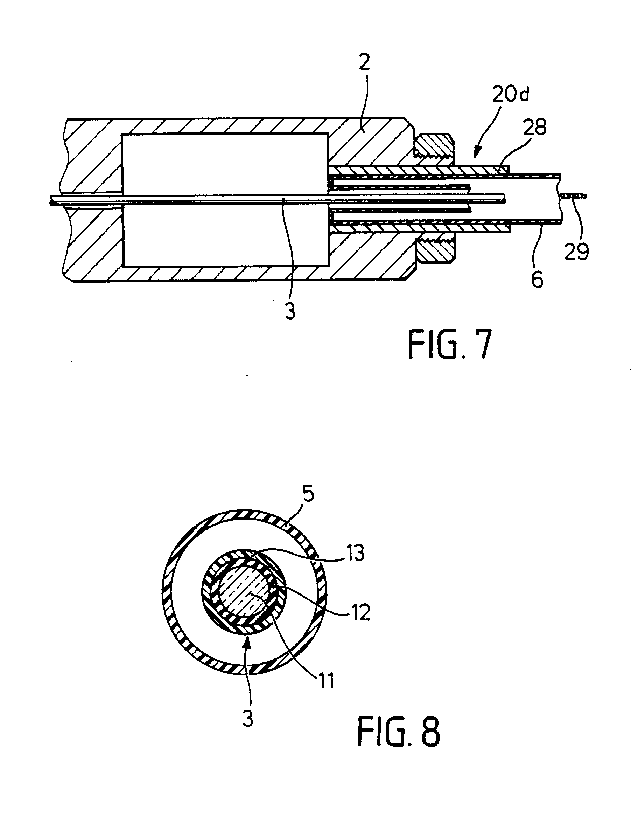

In Figur 8 ist der Aufbau der Lichtleitfaser 3 gezeigt, die aus einem Lichtleitkern II aus Quarz-glas mit einem Durchmesser von 600 $\mu$, einer darauf aufgebrachten weichen Silikonschicht I2 mit einer Schichtdicke von 50 bis I00 $\mu$ und einem diese umgebenden relativ harten Mantel I3 aus Polytetrafluorethylen mit einer Manteldicke von etwa 200 $\mu$ aufgebaut ist. Die Lichtleitfaser 3 ist in dem Befestigungsblock 7 in einer gestuften Boh-rung I4 eingespannt, deren Teilbohrung mit dem größeren Durchmesser die gesamte Lichtleitfaser 3 mit der Silikonschicht I2 und dem Mantel I3 auf-nimmt, während in die kurze vordere Teilbohrung lediglich der von Silikonschicht und Mantel befreite Lichtleitkern II eingeführt ist. In dieser Lage schließt die Stirnseite des Lichtleitkerns II mit der Stirnfläche des Befestigungsblockes 7 ab. In die Stirnseite des Lichtleitkerns II wird mit Hilfe einer Fokussierlinse I5 am vorderen Ende des Einkoppelstücks 2 Laserstrahlung in die Lichtleitfaser 3 eingekoppelt.

Auf der anderen Seite ist die Lichtleitfaser 3 in dem Einsatzstück 8 ebenfalls in einer gestuften Bohrung I6 gehalten. Das Einsatzstück 8 ist in der Teilbohrung mit dem größeren Durchmesser als Spreizhülse mit Längsschlitzen I7 ausgebildet und umfaßt die Lichtleitfaser mitsamt dem Mantel I3.Beim Aufstecken des Hüllschlauches 5 auf die Spreizhülse wird die Lichtleitfaser 3 eingespannt. In der vorderen Teilbohrung mit geringerem Durch-messer ist der Lichtleitkern II gelegen, von dem Silikonschicht und Mantel entfernt worden sind. Diese vordere Teilbohrung umgibt den Lichtleitkern II mit Abstand, so daß durch den Hüllschlauch 5 transportiertes Gas durch die Längsschlitze I7 in die vordere Bohrung geführt wird und dort das freie Ende des Lichtleitkerns II kühlt.

Bei starkem Abbiegen oder Aufwickeln der Lichtleitfaser 3 kann der Lichtleitkern II aus dem Befestigungsblock 7 oder dem Einsatzstück 8 herausgedrückt werden, wie dies in den Figuren 2 und 3 gestrichelt dargestellt ist. Um dieses zu verhindern, ist in dem Einsatzstück 2 für den Hüllschlauch 5 ein Längenausgleichselement 20 vorgesehen, das längenveränderlich , radial jedoch nicht dehnbar ist.

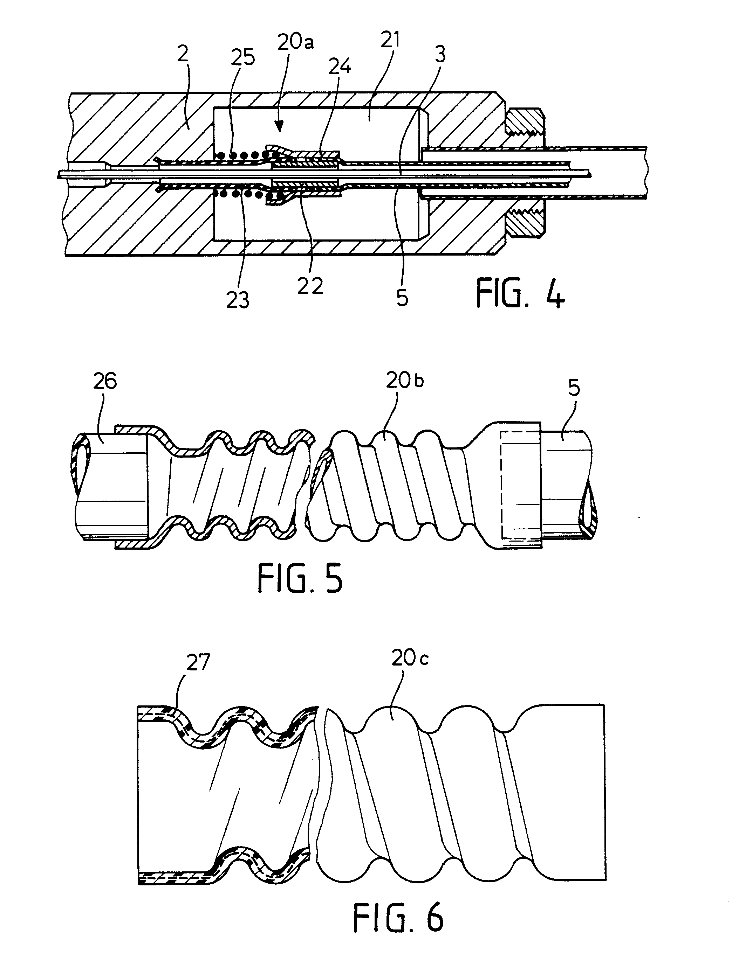

Eine Ausführungsform des Längenausgleichselementes ist in Figur 4 dargestellt und insgesamt mit 20a bezeichnet. Dieses Längenausgleichselement 20a ist in einem hinteren Hohlraum 21 des Einkoppelstückes 2 gelegen. In diesem Hohlraum endet der innere Hüllschlauch 5 und ist auf eine, die Lichtleitfaser 3 mit Abstand umgebende Hülse aufgesteckt und dort verklebt. Auf die andere Seite der Hülse ist ein elastischer Silikonschlauch aufgesteckt, der seinerseits in dem Einkoppelstück 2 befestigt ist. Dieser Silikonschlauch 23 dient somit praktisch als Endabschnitt des inneren Hüllschlauches 5.

Im Bereich der Hülse 22 ist auf die beiden Schläuche 5 und 23 eine Metallhülse aufgepreßt, die im Bereich des elastischen Silikonschlauches 23 aufgeweitet ist. Zwischen dem Gehäuse des Einkoppelstückes 2 und dieser Aufweitung ist eine Spiralfeder 25 gelegen, die den elastischen Silikonschlauch 23 eng umgibt. Die Spiralfeder 25 ist auf ihrer einen Seite mit dem Gehäuse des Einkoppelstückes 2 verlötet. Die Spiralfeder 25 ist in der Ruhestellung so gewickelt, daß sie sich sowohl verlängern als auch verkürzen kann. Die Spiralfeder verhindert ein radiales Ausbeulen des elastischen Silikonschlauches, wenn durch diesen das Gas zur Kühlung der Lichtleitfaser geleitet wird. Wenn jetzt bei einer starken Biegung bzw. beim Aufwickeln der Lichtleitfaser Längenänderungen zwischen dem Hüllschlauch 5 und der Lichtleitfaser auftreten, so werden diese durch das Längenausgleichselement 20a kompensiert. Zugkräfte auf die Lichtleitfaser treten daher nicht auf.

In Figur 5 ist eine weitere Ausführungsform eines Längenausgleichselementes dargestellt und mit 20b bezeichnet. Dieses Längenausgleichselement besteht aus einem metallischen Wellschlauch, der die Funktionen des oben erwähnten elastischen Silikonschlauches 23 und der Spiralfeder 25 übernimmt. In die eine Seite des Wellschlauches 20b ist der innere Hüllschlauch 5 eingesetzt, die andere Seite ist gegebenenfalls über einen hier angedeuteten Schlauch 26 mit dem Gehäuse des Einkoppelstückes verbunden.

In Figur 6 ist ein weiteres Längenausgleichselement 20c gezeigt, das ebenfalls aus einem Wellschlauch besteht, der jedoch in diesem Falle aus Kunststoff ist, in den zur Armierung und zur Verhinderung der radialen Dehnung Fäden oder ein Gewebe 27 eingelegt sind.

In Figur 7 ist ein Längenausgleichselement 20d in diesem Falle für den äußeren Hüllschlauch 6 dargestellt. Dieses Längenausgleichselement 20d weist eine Teleskophülse 28 auf, die am Ende des Einkoppelstücks 2 in diesem befestigt ist und den äußeren Hüllschlauch 6 glatt umgibt. Dieser äußere Hüllschlauch 6 ist in der Teleskophülse 28 verschiebbar, so daß Längenänderungen zwischen Lichtleitfaser 3 und Hüllschlauch 6 ausgeglichen werden können. Um ein Herausziehen des Hüllschlauches 6 aus der Teleskophülse zu vermeiden, sind entweder geeignete Anschläge vorgesehen oder längs des Hüllschlauches 6 ist ein Stahlseil 29 verlegt, dessen eines Ende am Einkoppelstück und dessen anderes Ende am hier nicht gezeigten freien Ende der Lichtleitfaser 3 befestigt ist. Die Länge des Stahlseils 29 entspricht der Länge der Lichtleitfaser zwischen den beiden Befestigungspunkten.

**Ansprüche**

1. Lichtleiter, insbesondere für medizinische Instrumente, mit einer Lichtleitfaser, einer die Lichtleitfaser mit Abstand umgebenden Hülle aus mindestens einem Hüllschlauch und einem Einkoppelstück auf einer Seite des Lichtleiters zum Einkoppeln von Lichtstrahlung in die Lichtleitfaser, wobei das eine Ende der Lichtleitfaser und der Hüllschlauch in dem Einkoppelstück und das andere Ende der Lichtleitfaser in dem Hüllschlauch eingespannt sind, dadurch gekennzeichnet, daß in den Hüllschlauch (5, 6) ein axial längenveränderliches, radial jedoch im wesentlichen nicht dehnbares Längenausgleichselement (20, 20a bis 20d) eingesetzt ist.

2. Lichtleiter nach Anspruch 1, dadurch gekennzeichnet, daß das Längenausgleichselement (20a) einen elastischen Schlauch (23) aufweist, der von einer Spiralfeder (25) umgeben ist.

3. Lichtleiter nach Anspruch 1, dadurch gekennzeichnet, daß das Längenausgleichselement ein Wellschlauch (20b, 20c) ist.

4. Lichtleiter nach Anspruch 3, dadurch gekennzeichnet, daß der Wellschlauch (20b) aus Metall ist.

5. Lichtleiter nach Anspruch 3, dadurch gekennzeichnet, daß der Wellschlauch (20c) aus Kunststoff ist, in den ein Gewebe oder Fäden (27) eingebettet sind.

6. Lichtleiter nach Anspruch 1, dadurch gekennzeichnet, daß das Längenausgleichselement (20d) als Teleskopsystem (28, 6) ausgebildet ist und eine Teleskophülse (28) aufweist, in der ein Hüllschlauch (6) der Hülle (4) gleitet.

FIG.1

FIG. 2

FIG. 3

FIG. 4

FIG. 5

FIG. 6

FIG. 7

FIG. 8